# EUROPEAN PATENT APPLICATION

(11) **EP 4 349 341 A1**
(43) Date of publication of application: **10.04.2024**
(21) Application number: 22816449.7
(22) Date of filing: 31.05.2022
(51) Int. Cl.: A61K 31/506, A61P 11/00, A61P 43/00

(54) **PHARMACEUTICAL PREPARATION FOR PREVENTING OR TREATING PULMONARY FIBROSIS**

(30) Priority: 01.06.2021 KR 20210071130
(71) Applicant: J2H Biotech Inc., Suwon-Si, Gyeonggi-do 16648 (KR)
(72) Inventor: RYU, Hyung-Chul, Hwaseong-si, Gyeonggi-do 18526 (KR); KIM, Jae-Sun, Suwon-si, Gyeonggi-do 16658 (KR); LIM, Jee-Woong, Seongnam-si, Gyeonggi-do 13600 (KR); KWON, Soon-Jin, Osan-si, Gyeonggi-do 18148 (KR); LEE, Seung-Yong, Suwon-si, Gyeonggi-do 16410 (KR)
(74) Representative: Maiwald GmbH
(86) International application number: PCT/KR2022/007778
(87) International publication number: WO 2022/255790

(57) **Abstract**

The present disclosure relates to medical use of a compound of chemical formula 1 for treatment or prevention of pulmonary fibrosis. The present disclosure also relates to combination therapy with the compound of chemical formula 1 and nintedanib.

## Description

### [Technical Field]

This application claims priority to Korean Patent Application No. 10-2021-0071130 filed on June 1, 2021, and all contents disclosed in the specification and drawings of the application are incorporated in this application by reference.

The present disclosure relates to pharmaceutical preparations for treating or preventing pulmonary fibrosis. That is, the present disclosure relates to the medical use of a compound for treating or preventing pulmonary fibrosis. The present disclosure also relates to combination preparations, i.e. combination therapy, for the treatment or prevention of pulmonary fibrosis.

### [Background Art]

Pulmonary fibrosis is a disease in which the lungs become increasingly hard and function deteriorates, leading to death due to difficulty breathing. Causes include rheumatic diseases, radiation exposure, and mold exposure, and there is also idiopathic pulmonary fibrosis, for which no specific cause can be found.

Cases of lung tissue damage and fibrosis due to radiation exposure during chemotherapy or in industrial settings are very frequent. For example, 10 to 15% of patients who receive chest radiation therapy develop radiation pneumonitis after 2 to 3 months, and develop fibrotic disease, a chronic side effect, after 6 months. Fibrosis that has progressed in this way is irreversible and cannot be recovered.

The basic treatment for pulmonary fibrosis is taking drugs that slow down the progression of fibrosis as much as possible, and in severe cases, lung transplantation should be considered. There are two prescription drugs for pulmonary fibrosis currently in clinical use: pirfenidone and nintedanib.

Meanwhile, in the case of combination therapy, in which two drugs are administered in combination, it is practically impossible to predict the effect when used together. However, such combination administration has several advantages, such as lowering the dosage of each drug, so research on combination preparations is needed.

### [Disclosure]

### [Technical Problem]

The present disclosure provides a pharmaceutical preparation useful for preventing or treating pulmonary fibrosis. Another aspect of the present disclosure is to provide a method for preventing or treating pulmonary fibrosis.

### [Technical Solution]

In order to achieve the above object, an embodiment of the present disclosure provides a pharmaceutical preparation for preventing or treating pulmonary fibrosis, comprising as an active agent a pyrimidine-4-carboxamide compound of the following chemical formula 1:

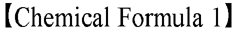

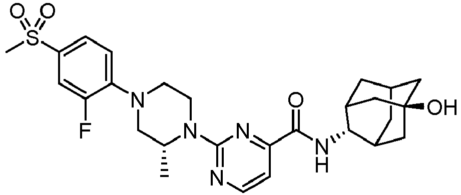

While trying to develop a medicine for the prevention or treatment of pulmonary fibrosis, the present inventors have discovered that the compound (2-((R)-4-(2-fluoro-4-(methylsulfonyl)phenyl)-2-methylpiperazin-1-yl)-N-((1R,2s,3S,5S,7S)-5-hydroxyadamantan-2-yl)pyrimidine-4-carboxamide) represented by Chemical Formula 1 was very effective in alleviating pulmonary fibrosis, and through this, the present invention was completed.

The above compound is a compound included in the chemical formula disclosed in the PCT Application Publication WO2011-139107. The compound is known as a 11β-HSD1 (11β-hydroxysteroid dehydrogenase type 1) enzyme inhibitor and is being studied primarily for use in treating metabolic diseases such as diabetes and non-alcoholic steatohepatitis. (PCT Application Publication WO2020-022708).

The compound of Chemical Formula 1 can be prepared by the method disclosed in the PCT Application Publication WO2011-139107.

The present disclosure also provides a method of treating, improving or preventing pulmonary fibrosis, characterized in that a therapeutically or prophylactically effective amount of the compound of Chemical Formula 1 is administered to a subject in need of treatment or prevention of pulmonary fibrosis. That is, the present disclosure provides the medical use of the compound of Chemical Formula 1 for the treatment or prevention of pulmonary fibrosis.

As used herein, the term "prevention" includes preventing recurrence, expansion, or development of pulmonary fibrosis in a patient.

As used herein, the term "treatment" includes eradicating, eliminating, or controlling pulmonary fibrosis and minimizing or delaying the expansion of pulmonary fibrosis.

In one embodiment of the present disclosure, pulmonary fibrosis is pulmonary fibrosis induced by drug therapy (eg, anticancer drug therapy) or radiation exposure. In another embodiment of the disclosure, the pulmonary fibrosis is idiopathic pulmonary fibrosis.

Another embodiment of the present disclosure provides a pharmaceutical preparation for preventing or treating pulmonary fibrosis, comprising the compound of Chemical Formula 1 as an active agent, and further comprising nintedanib (CAS number: 656247-17-5) or its pharmaceutically acceptable salt as a second active agent. That is, another embodiment of the present disclosure provides a combination preparation of the above two active agents for preventing or treating pulmonary fibrosis, that is, a combination therapy thereof.

The compound of Chemical Formula 1 and nintedanib synergistically increase the effect of treating or preventing pulmonary fibrosis.

The pharmaceutically acceptable salt of nintedanib may be an acid addition salt, and includes salts of the active compound prepared from relatively non-toxic acids. Acid-added salts can be obtained by contacting the neutral compounds with a sufficient amount of the desired acid and pure or inert solvent. Suitable pharmaceutically acceptable acid addition salts include salts derived from non-toxic organic acids including, but are not limited to, acetic acid, propionic acid, isobutyl acid, oxalic acid, maleic acid, malonic acid, benzoic acid, succinic acid, suberic acid, fumaric acid, mandelic acid, phthalic acid, benzenesulfonic acid, p-tolylsulfonic acid, citric acid, tartaric acid, methanesulfonic acid, and the like, and non-toxic inorganic acids including, but are not limited to, hydrochloric acid, hydrobromic acid, nitric acid, carbonic acid, monohydrogencarbonic acid, phosphoric acid, monohydrogenphosphric acid, dihydrogenphosphoric acid, sulfuric acid, monohydrogensulfuric acid, hydrogen iodide, phosphorous acid and the like. Also it includes a salt of amino acid such as arginate or its analogues, and it also includes analogues of organic acid such as glucuronic or galacturonic acid. For example, ethanesulfonic acid of nintedanib can be used.

As used herein, the term "compound of Chemical Formula 1" or "nintedanib" refers to the compound of Chemical Formula 1 and nintedanib, respectively, and is meant to include clathrates, hydrates, solvates, or polymorphs thereof.

As used herein, the term "polymorph" refers to solid crystalline forms of a compound of this disclosure or complex thereof. Different polymorphs of the same compound can exhibit different physical, chemical and/or spectroscopic properties. Different physical properties include, but are not limited to stability (e.g., to heat or light), compressibility and density (important in formulation and product manufacturing), and dissolution rates (which can affect bioavailability). Differences in stability can result from changes in chemical reactivity (e.g., differential oxidation, such that a dosage form discolors more rapidly when comprised of one polymorph than when comprised of another polymorph) or mechanical characteristics (e.g., tablets crumble on storage as a kinetically favored polymorph converts to thermodynamically more stable polymorph) or both (e.g., tablets of one polymorph are more susceptible to breakdown at high humidity). Different physical properties of polymorphs can affect their processing. For example, one polymorph might be more likely to form solvates or might be more difficult to filter or wash free of impurities than another due to, for example, the shape or size distribution of particles of it.

As used herein, the term "solvate" means the compound according to this disclosure that further includes a stoichiometric or non-stoichiometric amount of a solvent bound by non-covalent intermolecular forces. Preferred solvents are volatile, non-toxic, and acceptable for administration to humans in trace amounts.

As used herein, the term "hydrate" means the compound according to this disclosure that further includes a stoichiometric or non-stoichiometric amount of water bound by non-covalent intermolecular forces.

As used herein, the term "clathrate" means the compound according to this disclosure in the form of a crystal lattice that contains spaces (e.g., channels) that have a guest molecule (e.g., a solvent or water) trapped within.

If a compound (prodrug) is disassembled in the body and produces a compound of the present invention, such compound is also included within the scope of the present invention. As used herein and unless otherwise indicated, the term "prodrug" refers to a compound that has been hydrolyzed, oxidized and subjected to other reactions under biological conditions (*in vitro* or *in vivo*) to provide an active compound, especially the compound of the invention. Examples of prodrugs include, but are not limited to, biohydrolyzable amides, biohydrolyzable esters, biohydrolyzable carbamates, biohydrolyzable carbonates, and biohydrolyzable ureides, and compounds that contain biohydrolyzable moieties, such as biohydrolyzable phosphate analogs, that biohydrolyze to produce the compounds of the invention. Preferably, the prodrug of the compound having a carboxyl group functional group is a lower alkyl ester of a carboxylic acid. Carboxylic esters are usually formed by esterifying a portion of the carboxylic acid present in the molecule. Prodrugs can be readily prepared using well-known methods such as those described in Burger's Medicinal Chemistry and Drug Discovery 6th ed. (Donald J. Abrahamed., 2001, Wiley) and Design and Application of Prodrugs (H. Bundgaard ed., 1985, Harwood Academic Publishers Gmfb).

The compound of the present invention is generally administered in a therapeutically effective amount.

The compound(s) of the present invention can be administered by any suitable route in the form of a pharmaceutical composition adapted to such a route, and in a dose effective for the treatment intended. An effective dosage is typically in the range of about 0.01 to about 50 mg per kg body weight per day, preferably about 0.05 to about 20 mg/kg/day, in single or divided doses. Depending on age, species and disease or condition being treated, dosage levels below the lower limit of this range may be suitable. In other cases, still larger doses may be used without harmful side effects. Larger doses may also be divided into several smaller doses, for administration throughout the day. Methods for determining appropriate dosages are well known in the art.

When two active agents are administered according to the combination therapy of the present disclosure, the dosage of each active agent may be the same as the dosage when administered alone or may be reduced by 10-30% by weight compared to single administration. In one embodiment of the present disclosure, when used in combination, the compound of Chemical Formula 1 may be administered in an amount of 0.1 to 1 part by weight compared to the dosage of nintedanib. Nintedanib dosages are well known in the art (see Ofev^{®} dosages)

As previously described, when two active agents are administered, they may be administered simultaneously (in the same formulation or in separate formulations) or sequentially. Therefore, in one embodiment, the present disclosure provides a method for treating or preventing pulmonary fibrosis, comprising administering to a subject a therapeutically effective amount of the compound of Chemical Formula 1 and a therapeutically effective amount of the second active agent, nintedanib or a pharmaceutically acceptable salt thereof.

In one embodiment, one of the two active agents may be given in multiple doses, or both may be given in multiple doses. If not simultaneous, the timing between multiple doses can vary anywhere from more than 0 weeks to less than 20 weeks.

Additionally, combination methods, compositions and formulations are not limited to the use of just two agents; multiple treatment combinations are also envisioned. Dosage regimen for treating, preventing, or ameliorating a pathological condition may vary depending on various factors. These factors include the individual's age, weight, sex, diet, and medical condition, as well as any disorders the individual suffers from.

The pharmaceutical preparations that achieve the combination treatment disclosed herein are either in single dosage forms, optionally combined, or in separate dosage forms, primarily for simultaneous administration. In the present specification, the formulation may be a kit formulation containing two active agents separately. The pharmaceutical preparations that achieve the combination treatment of the present disclosure can also be administered sequentially as either of the agents administered by a regimen requiring two-step administration. A two-step dosing regimen may require sequential administration of the active agent or spaced administration of separate active agents. The time period between multiple administration steps can range from several minutes to several hours depending on the properties of each active agent such as potency, solubility, bioavailability, plasma half-life and kinetic profile of the active agent. Circadian variation in target molecule concentration is used to determine the optimal dosing interval.

In another aspect, another embodiment of the present disclosure provides a pharmaceutical preparation (combination preparation) comprising the compound of Chemical Formula 1 and a pharmaceutically acceptable carrier or additive; or a pharmaceutical preparation (combination preparation) comprising the compound of Chemical Formula 1, nintedanib or its pharmaceutically acceptable salt, and a pharmaceutically acceptable carrier or additive.

The term "pharmaceutically acceptable" means to be suitable for use as a pharmaceutical preparation, generally considered safe for such use, and officially approved for such use by a national governing body or to be listed in the Korean Pharmacopoeia or the United States Pharmacopoeia.

For the treatment of the disease or condition described above, the active agent or active agents described herein may be administered as follows: A plurality of active agents according to the present invention may be included together in one preparation below, or a plurality of active agents may be contained in separate preparations and taken together.

### Intranasal administration

The active agents according to the present invention may be administered nasally, and intranasal administration may be particularly preferable for the medical use of the present disclosure. In the present invention, such nasal administration includes conventional inhalant administration.

Such "intranasal administration" means delivery of a composition into the nose and nasal passages through either or both the nose or nasal passages, including delivery by a spray mechanism or droplet mechanism or delivery by aerosolization of the active agent. Administration of the composition by inhalation can be through the nose or mouth via delivery by a spray or droplet mechanism.

For nasal or inhalation delivery, the compositions of the present invention may be formulated by methods well known to those skilled in the art, for example, with representative solubilizing, diluting, or dispersing agents such as saline, preservatives such as benzyl alcohol, absorption accelerators, etc., but are not limited thereto. This liquid pharmaceutical composition may be prepared in the same manner as the oral administration composition described later.

Such intranasal administration can be performed using an intranasal delivery device already well known in the field to which the present invention pertains, and propellants such as fluorocarbons and hydrofluoroalkane may be used in these devices.

### Oral administration

The compounds of the present invention may be administered orally, including by swallowing, so that the compound enters the gastrointestinal tract, or absorbed into the blood stream directly from the mouth (e.g., buccal or sublingual administration).

Suitable compositions for oral administration include solid, liquid, gel or powder formulations, and have a dosage form such as tablet, lozenge, capsule, granule or powder.

Compositions for oral administration may optionally be enteric coated and may exhibit delayed or sustained release through the enteric coating. That is, the composition for oral administration according to the present invention may be a formulation having an immediate or modified release pattern.

Liquid formulations can include solutions, syrups and suspensions, which can be used in soft or hard capsules. Such formulations may include a pharmaceutically acceptable carrier, for example, water, ethanol, polyethylene glycol, cellulose, or an oil. The formulation may also include one or more emulsifying agents and/or suspending agents.

In a tablet dosage form the amount of drug, active ingredient, present may be from about 0.05% to about 95% by weight, more typically from about 2% to about 50% by weight of the dosage form. In addition, tablets may contain a disintegrant, comprising from about 0.5% to about 35% by weight, more typically from about 2% to about 25% of the dosage form. Examples of disintegrants include, but are not limited to, lactose, starch, sodium starch glycolate, crospovidone, croscarmellose sodium, maltodextrin, or mixtures thereof.

Suitable lubricants, for use in a tablet, may be present in amounts from about 0.1% to about 5% by weight, and include, but are not limited to, talc, silicon dioxide, stearic acid, calcium, zinc or magnesium stearate, sodium stearyl fumarate and the like.

Suitable binders, for use in a tablet, include, but are not limited to, gelatin, polyethylene glycol, sugars, gums, starch, polyvinyl pyrrolidone, hydroxypropyl cellulose, hydroxypropylmethyl cellulose and the like. Suitable diluents, for use in a tablet, include, but are not limited to, mannitol, xylitol, lactose, dextrose, sucrose, sorbitol, microcrystalline cellulose and starch.

Suitable solubilizers, for use in a tablet, may be present in amounts from about 0.1% to about 3% by weight, and include, but are not limited to, polysorbates, sodium lauryl sulfate, sodium dodecyl sulfate, propylene carbonate, diethyleneglycol monoethyl ether, dimethyl isosorbide, polyethylene glycol (natural or hydrogenated) castor oil, HCOR^{™} (Nikkol), oleyl ester, Gelucire^{™}, caprylic/caprylic acid mono/diglyceride, sorbitan fatty acid esters, and Solutol HS^{™}.

### Parenteral Administration

Compounds of the present disclosure may be administered directly into the blood stream, muscle, or internal organs. Suitable means for parenteral administration include intravenous, intramuscular, subcutaneous intraarterial, intraperitoneal, intrathecal, intracranial, and the like. Suitable devices for parenteral administration include injectors (including needle and needle-free injectors) and infusion methods.

Compositions for parenteral administration may be formulated as immediate or modified release, including delayed or sustained release.

Most parenteral formulations are liquid compositions, and the liquid composition is an aqueous solution containing the active ingredient according to the present invention, a salt, a buffering agent, an isotonic agent, and the like.

Parenteral formulations may also be prepared in a dehydrated form (e.g., by lyophilization) or as sterile non-aqueous solutions. These formulations can be used with a suitable vehicle, such as sterile water. Solubility-enhancing agents may also be used in preparation of parenteral solutions.

### Topical Administration

Compounds of the present invention may be administered topically to the skin or transdermally. Formulations for this topical administration can include lotions, solutions, creams, gels, hydrogels, ointments, foams, implants, patches and the like. Pharmaceutically acceptable carriers for topical administration formulations can include water, alcohol, mineral oil, glycerin, polyethylene glycol and the like. Topical administration can also be performed by electroporation, iontophoresis, phonophoresis and the like.

Compositions for topical administration may be formulated as immediate or modified release, including delayed or sustained release.

### [Advantageous Effects]

The present disclosure provides a pharmaceutical preparation useful for treating or preventing pulmonary fibrosis, comprising the compound of Chemical Formula 1 as an active agent. That is, the present disclosure provides a medical use of the compound of Chemical Formula 1, which is useful for the treatment or prevention of pulmonary fibrosis.

The present disclosure also provides a pharmaceutical preparation useful for treating or preventing pulmonary fibrosis, comprising two active agents: the compound of Chemical Formula 1 and nintedanib or its pharmaceutically acceptable salt. That is, the present disclosure provides a combination therapy that exhibits a synergistic effect and is useful for the treatment or prevention of pulmonary fibrosis.

### [Brief Description of Figures]

Figure 1 shows the results of Masson's trichrome staining of lung tissue at the irradiation site when each test compound was orally administered to a pulmonary fibrosis mouse model induced by radiation.
Figure 2 is a graph quantifying the degree of collagen deposition using an image program after photographing lung tissue at the radiation site under a microscope when each test compound was orally administered to a pulmonary fibrosis mouse model induced by radiation.
Figure 3 is a graph quantifying the mRNA expression of the col1a1 gene using qPCR method with extracting RNA from lung tissue when each test substance was orally administered to a bleomycin-induced pulmonary fibrosis mouse model.
Figure 4 is a graph quantifying the mRNA expression of the TGFb gene using qPCR method with extracting RNA from lung tissue when each test compound was orally administered to a bleomycin-induced pulmonary fibrosis mouse model.
Figure 5 is a graph quantified by measuring the amount of hydroxyproline using an ELISA method after obtaining a lysate by dissolving lung tissue when each test compound was orally administered to a bleomycin-induced pulmonary fibrosis mouse model.

### [Mode for Invention]

Hereinafter, the present invention is described in considerable detail with examples to help those skilled in the art understand the present invention. However, the following examples are offered by way of illustration and are not intended to limit the scope of the invention. It is apparent that various changes may be made without departing from the spirit and scope of the invention or sacrificing all of its material advantages.

Reference Example 1. Administration to an animal model in which pulmonary fibrosis is induced (irradiation)

The test compound was administered orally to C57BL/6 male 8-week-old mice, and 1 hour later, radiation (90Gy, 4 mm collimator) was irradiated to the chest at a size of 3 mm. Afterwards, test compounds were administered orally twice a day for 14 days, and then an autopsy was performed. Mouse lung tissue was fixed with 10% formalin, paraffin sections were made, and the degree of collagen deposition within the tissue was confirmed using trichrome staining. The test groups were as shown in Table 1 below.

**[Table 1]**

| Test Group | Test Substance | Single Dose | Administration Route | Number of Administration |
|---|---|---|---|---|
| G1 | Vehicle | - | Oral | Twice a day, 14 days |
| G2 | Nintedanib | 30 mg/kg | Oral | Twice a day, 14 days |
| G3 | Chemical Formula 1 Compound | 10 mg/kg | Oral | Twice a day, 14 days |
| G4 | Chemical Formula 1 Compound + nintedanib | 10 + 30 mg/kg | Oral | Twice a day, 14 days |

### Reference Example 2. Administration to an animal model in which pulmonary fibrosis is induced (bleomycin injection)

C57BL/6N mice were anesthetized using isoflurane. Afterwards, an incision was made in the center of the neck to expose the airway. Bleomycin dissolved in physiological saline injection solution was slowly injected at 1.25 mg/kg into the exposed upper respiratory tract using an insulin syringe. Afterwards, the insulin syringe was removed and the incision was sutured. Afterwards, test compounds were administered orally twice a day for 14 days, and then an autopsy was performed. The mouse's left lung tissue was fixed with 10% formalin, paraffin sections were made, and the degree of lung fibrosis was evaluated using H&E staining. The right lung was tissue lysed for qPCR and hydroxyproline measurements. Afterwards, it was used in each experiment to evaluate the expression of lung fibrosis-related genes and the degree of collagen deposition in the lung. The test groups were as shown in Table 2 below.

**[Table 2]**

| Test Group | Test Substance | Single Dose | Administration Route | Number of Administration |
|---|---|---|---|---|
| G1 | Vehicle (Normal Group) | - | Oral | Twice a day, 14 days |
| G2 | Vehicle (Induced Group) | - | Oral | Twice a day, 14 days |
| G3 | Chemical Formula 1 Compound | 30 mg/kg | Oral | Twice a day, 14 days |
| G4 | Chemical Formula 1 Compound | 100 mg/kg | Oral | Twice a day, 14 days |
| G5 | Chemical Formula 1 Compound | 200 mg/kg | Oral | Twice a day, 14 days |
| G6 | Chemical Formula 1 Compound + nintedanib | 60 + 30 mg/kg | Oral | Twice a day, 14 days |
| G7 | Nintedanib | 60 mg/kg | Oral | Twice a day, 14 days |

### Example 1. Antifibrotic efficacy of the compound of Chemical Formula 1 in an animal model of pulmonary fibrosis

According to Reference Example 1, test compounds were administered to a mouse model of radiation-induced pulmonary fibrosis and an autopsy was performed. Mouse lung tissue was fixed in 10% neutral formalin for one day and paraffin sections were made. To remove paraffin around the tissue, it was sequentially reacted in xylene, 95, 90, and 70% ethanol solutions for 5 minutes each. To activate the tissue antigen, the tissue was immersed in a 0.1 M citric acid (pH 6.0) solution and boiled for 20 minutes. Then, it was sequentially reacted with Bouin's solution for 1 minute, Weigert's hematoxylin for 10 minutes, Phosphotunstic/phosphomolydic acid for 10 minutes, aniline blue for 5 minutes, and 1% acetic acid for 1 minute. Next, after a dehydration process, it was sealed with a cover glass and observed under a microscope (Carl Zeiss Vision).

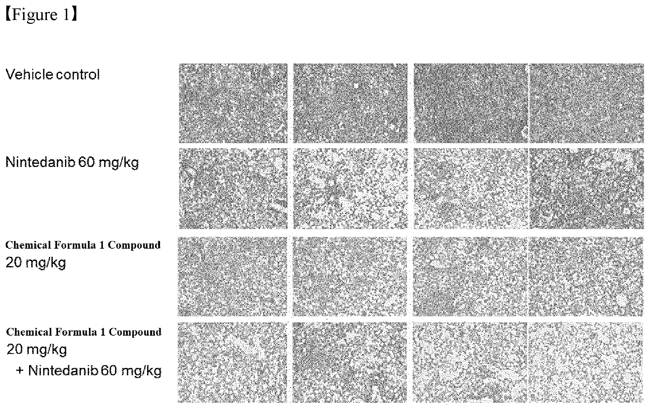

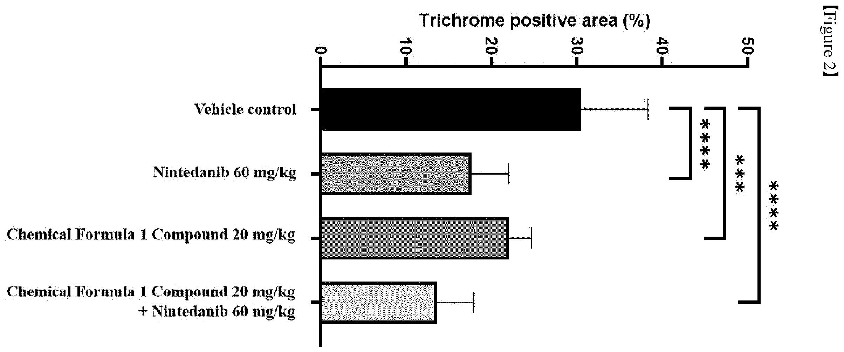

As a result of the test, as shown in Figures 1 and 2, the anti-fibrotic effect of suppressing collagen deposition was confirmed in all test compound administration groups compared to the vehicle group (G1). Among these, the test groups showing high significance were the group administered nintedanib alone (G2) and the group administered in combination with nintedanib and the compound of Chemical Formula 1 (G4). In particular, the group administered in combination with nintedanib and the compound of Chemical Formula 1 (G4) showed better efficacy compared to the group administered nintedanib (G2).

In conclusion, the compound of Chemical Formula 1 significantly inhibits collagen deposition, which is the primary evaluation index of pulmonary fibrosis, and in particular, when administered in combination with nintedanib, the effect was confirmed to be superior to that of nintedanib administered alone.

### Example 2. Antifibrotic efficacy of the compound of Chemical Formula 1 in an animal model of pulmonary fibrosis

According to Reference Example 2, test compounds were administered to a mouse model of bleomycin-induced pulmonary fibrosis and an autopsy was performed. The left lung tissue of the mouse was fixed in 10% neutral formalin for one day and paraffin sections were made. To remove paraffin around the tissue, it was sequentially reacted in xylene, 95, 90, and 70% ethanol solutions for 5 minutes each. Next, for trichrome staining, the cells were sequentially reacted with Bouin's solution for 1 minute, Weigert's hematoxylin for 10 minutes, Phosphotunstic/phosphomolydic acid for 10 minutes, aniline blue for 5 minutes, and 1% acetic acid for 1 minute. Next, after a dehydration process, it was sealed with a cover glass and observed under a microscope (Carl Zeiss Vision). Half of the mouse's right lung tissue was lysed for qPCR, RNA was extracted, and cDNA was synthesized. Afterwards, gene expression analysis of Col1a1, an extracellular matrix that causes fibrosis, and TGFβ, an important cytokine that causes fibrosis, was performed using qPCR equipment. The other half of the tissue was lysed and the lysate was analyzed using a hydroxyproline ELISA kit.

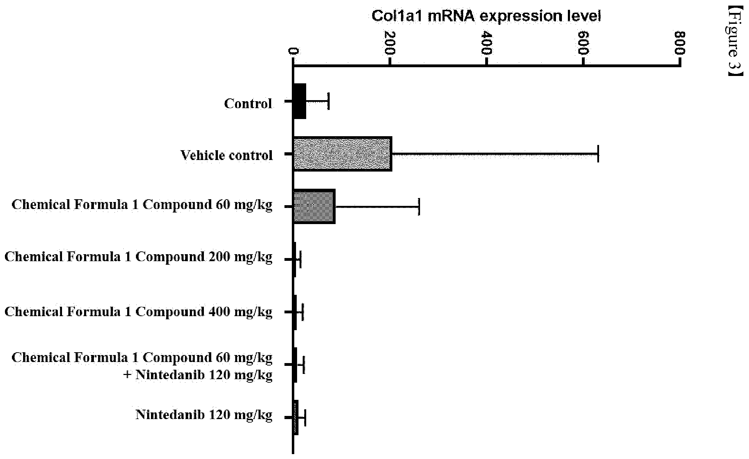

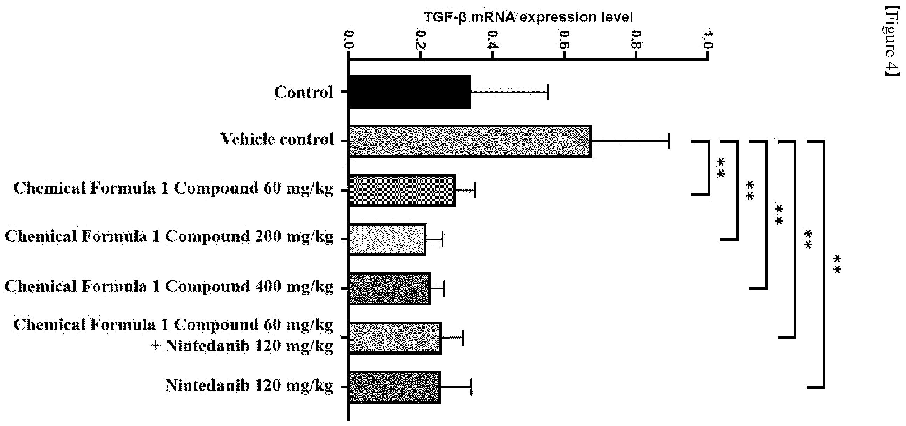

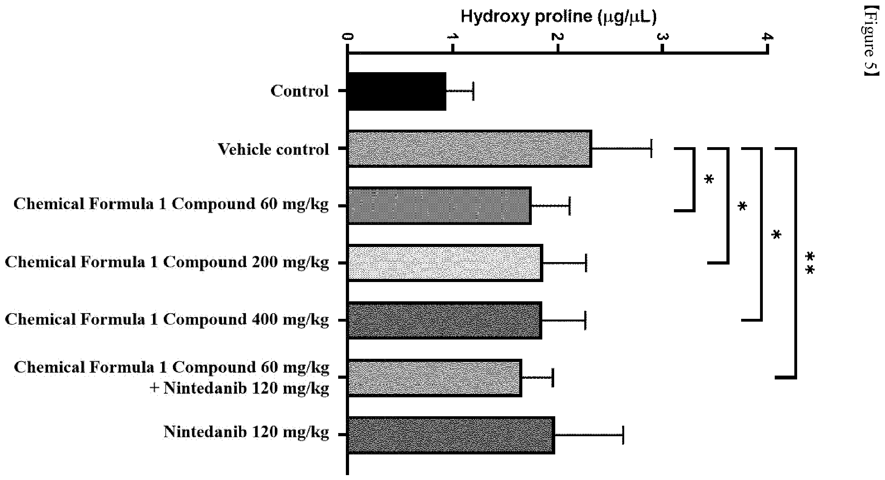

As a result of the test, in Figure 3, when comparing Col1a1 gene expression in lung tissue, no statistically significant difference was observed, but a decreasing trend was confirmed in all administration groups compared to the vehicle administration group (G2). In addition, when comparing the gene expression of TGFβ in Figure 4, significant differences were confirmed in all administration groups compared to the vehicle administration group (G2). In Figure 5, as a result of confirming the expression of hydroxyproline in lung tissue, significant differences were confirmed in all administration groups except the nintedanib single administration group (G7) compared to the vehicle administration group (G2). Especially better results were confirmed in the combined administration group of nintedanib and the compound of Formula 1 (G6).

In conclusion, the compound of Chemical Formula 1 significantly inhibits collagen deposition, which is the primary evaluation index of pulmonary fibrosis, and in particular, when administered in combination with nintedanib, the effect was confirmed to be superior to that of nintedanib administered alone.

## Claims

1. A pharmaceutical preparation for preventing or treating pulmonary fibrosis, comprising the compound of Chemical Formula 1 as an active agent.

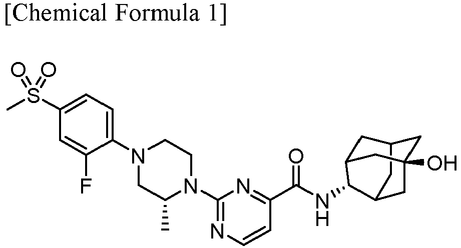

2. The pharmaceutical preparation of claim 1, wherein the pulmonary fibrosis is caused by drug therapy or radiation exposure.

3. The pharmaceutical preparation of claim 1, wherein the pulmonary fibrosis is idiopathic pulmonary fibrosis.

4. The pharmaceutical preparation of any one of claims 1 to 3, wherein the preparation further comprises nintedanib or its pharmaceutically acceptable salt as an active agent.

5. A method for treating or preventing pulmonary fibrosis, comprising administering a therapeutically or prophylactically effective amount of the compound of Chemical Formula 1 to a subject in need of treatment or prevention of pulmonary fibrosis.

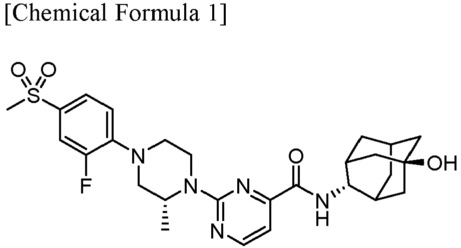

6. The method of claim 5, wherein the pulmonary fibrosis is caused by drug therapy or radiation exposure.

7. The method of claim 5, wherein the pulmonary fibrosis is idiopathic pulmonary fibrosis.

8. The method of any one of claims 5 to 7, wherein the method further comprises administering a therapeutically or prophylactically effective amount of nintedanib or its pharmaceutically acceptable salt to the subject.
